# FASCICULE DE BREVET EUROPEEN

(11) **EP 3 478 151 B1**
(45) Date de publication et mention de la délivrance du brevet: **10.05.2023**
(21) Numéro de dépôt: 17732468.8
(22) Date de dépôt: 28.06.2017
(51) Int. Cl.: A61B 3/00, A61B 3/10, A61B 3/14, A61B 5/00, G02B 27/01

(54) **DISPOSITIF DE MESURE OCULAIRE ÉQUIPÉ D'UN SYSTÈME OPTIQUEMENT ALIGNÉ SUR L'AXE DE VISION DE L'UTILISATEUR**
AUGENMESSVORRICHTUNG MIT EINEM AUF DIE BLICKRICHTUNG DES BENUTZERS OPTISCH AUSGERICHTETEN SYSTEM
OCULAR MEASUREMENT DEVICE EQUIPPED WITH A SYSTEM OPTICALLY ALIGNED ON THE LINE OF SIGHT OF THE USER

(30) Priorité: 29.06.2016 FR 1656069
(43) Date de publication de la demande: 08.05.2019
(73) Titulaire: Institut Mines Telecom, 29238 Brest Cedex 3 (FR)
(72) Inventeur: DE BOUGRENET DE LA TOCNAYE, Jean-Louis, 29820 Guilers (FR); NOURRIT, Vincent, 29200 Brest (FR); FERRAGUT, Stéphanie, 29200 Brest (FR)
(74) Mandataire: Cabinet Nony
(86) Numéro de dépôt international: PCT/EP2017/065948
(87) Numéro de publication internationale: WO 2018/002110

(56) Documents cités:
- JP-A- 2001 066 543
- JP-B1- 5 824 697
- JP-B2- 3 847 799
- US-A1- 2013 010 097

## Description

### 1. DOMAINE DE L'INVENTION

L'invention concerne le domaine des appareils de mesure des mouvements oculaires, aussi appelés oculomètres ou dispositifs oculométriques.

L'invention concerne plus particulièrement un dispositif d'oculométrie portatif destiné notamment au traitement orthoptique. Un tel dispositif comprend une monture adaptée pour être portée par un utilisateur et un système embarqué sur cette monture dédié à la mesure des mouvements oculaires de l'utilisateur.

L'orthoptie se définit comme étant l'ensemble des processus couvrant le dépistage, la rééducation, la réadaptation et l'exploration fonctionnelle des troubles de la vision.

### 2. ARRIÈRE-PLAN TECHNOLOGIQUE

L'oculométrie est utilisée comme technique de mesure des mouvements des yeux dans de nombreux domaines d'application : recherche en psychologie, marketing (mesure du regard pour déterminer l'intérêt des consommateurs de produits ou utilisateurs de services), réalité virtuelle, réalité augmentée, navigation informatique (interface homme-machine), médical, rééducation oculomotrice, etc.

Les oculomètres sont des dispositifs permettant d'enregistrer et de contrôler les mouvements des yeux. Traditionnellement, ces dispositifs comprennent, pour un oeil donné, une source de lumière infrarouge destinée à illuminer au moins partiellement ledit oeil donné et un capteur d'image destiné à enregistrer des images dudit oeil donné. Une unité de traitement coopère généralement avec le capteur d'image pour étudier, par analyse d'image, le comportement oculaire de l'utilisateur.

La réduction des coûts des capteurs combinée à la forte augmentation de l'intégration des composants électroniques et optiques dans les systèmes, a poussé ces dernières années les chercheurs à mettre au point des solutions oculométriques embarquées. Une tendance accentuée par l'augmentation de la puissance de calcul des ordinateurs et la diversité des domaines concernés. Ces solutions oculométriques se composent principalement d'un support destiné à être porté par un utilisateur, tel qu'un casque ou une monture de lunettes par exemple, et d'un système optique embarqué sur ce support.

Une dispositif d'oculométrie classique, tel que celui décrit dans le document de brevet US 2015/0061996, comprend une monture de lunettes configurée pour recevoir deux éléments optiques au moins partiellement transparents sur chacun desquels est monté, face interne, un élément de support optique. Cet élément de support optique, pour un oeil donné, est conçu pour supporter une source de lumière ainsi qu'au moins un capteur d'image, ces éléments étant disposés de manière excentrique par rapport à l'axe de vision de cet oeil donné afin de ne pas perturber la vision naturelle. La source de lumière et le capteur d'image sont dirigés vers l'œil de l'utilisateur et destinés respectivement à éclairer uniformément l'œil et à capturer la lumière réfléchie par cet oeil. Dans d'autres solutions connues, la source et le capteur d'image peuvent être disposés directement sur la monture (par des moyens de liaison mécanique ou encapsulés dans la monture pour éviter un porte-à-faux) et orientés face aux yeux de l'utilisateur.

US 2013/010097 divulgue un dispositif de poursuite des mouvements des yeux, comprenant une monture de lunettes, un LED infrarouge pour illuminer les yeux, un capteur d'images agencé sur la face externe de la monture et un élément de visualisation des yeux positionné dans le champ de vision et réfléchissant la lumière infrarouge réfléchie par les yeux vers le capteur d'images.

L'inconvénient principal de ce genre de dispositif est la proximité et l'excentricité à la fois du capteur d'image et de la source par rapport à l'œil de l'utilisateur.

Pour répondre à des contraintes d'encombrement et d'intégration, le capteur d'image doit en effet combiner courte focale, large champ de captation, grande ouverture numérique et l'excentrement, nécessitant l'intégration d'un système optique complexe et coûteux. Par ailleurs, ce type de système optique génère un certain nombre d'aberrations optiques (comme les distorsions géométriques typiquement), qui doivent être prises en compte lors du traitement postérieur des images capturées, ce qui rend le processus d'acquisition et de traitement d'image complexe et long, et potentiellement moins performant. Enfin, cette configuration entraîne une déformation « naturelle » de la pupille du fait de la proximité de la source de lumière par rapport à l'œil de l'utilisateur.

La source de lumière est constituée généralement d'un ensemble de diodes électroluminescentes (ou LED pour *«Light-Emitting Diode* ») disposées sur la monture afin de créer un éclairage uniforme de la cornée quelle que soit la direction du regard. Les diodes électroluminescentes, de petites tailles, sont facilement intégrables mais couvrent un champ d'émission relativement étroit (par exemple 40°). Leur orientation dans la monture est donc critique pour assurer une illumination uniforme, d'autant plus que l'éclairage est excentré par rapport à l'axe de vision (pour ne pas perturber la vision naturelle), ce qui n'est pas optimal.

### 3. OBJECTIFS DE L'INVENTION

L'invention, dans au moins un mode de réalisation, a notamment pour objectif de pallier ces différents inconvénients de l'état de la technique.

Plus précisément, dans au moins un mode de réalisation de l'invention, un objectif est de fournir un dispositif d'oculométrie, équipé d'un système embarqué, de mise en oeuvre simple et peu coûteuse.

Au moins un mode de réalisation de l'invention a également pour objectif de fournir un tel dispositif offrant la possibilité d'utiliser des optiques moins complexes et de meilleure qualité, afin de produire une imagerie de meilleure qualité (et donc de simplifier le traitement d'image à réaliser postérieurement).

### 4. EXPOSÉ DE L'INVENTION

Dans un mode de réalisation particulier de l'invention, il est proposé un dispositif d'oculométrie comprenant :
- une monture adaptée pour être portée par un utilisateur, la monture ayant une face interne dirigée vers les yeux de l'utilisateur et une face externe opposée à la face interne,
- un système embarqué sur la monture, comprenant, pour au moins un oeil donné dudit utilisateur :
   * une source de lumière destinée à illuminer au moins partiellement ledit oeil de façon à générer une lumière réfléchie par ledit oeil donné, et
   * un capteur d'image destiné à capturer la lumière réfléchie par ledit oeil donné, ledit capteur d'image étant disposé spatialement hors axe par rapport un axe de référence correspondant à un axe de vision dudit oeil lorsque la monture est portée par ledit utilisateur,
ledit capteur d'image étant disposé sur la face externe de la monture et possède un axe optique dirigé vers l'extérieur de la monture, et le système embarqué comprenant des moyens optiques semi-réfléchissants disposés sur la face externe de la monture et, pour un oeil donné, configurés par rapport à l'axe de référence dudit oeil donné pour réfléchir directement vers l'axe optique du capteur d'image la lumière réfléchie par ledit oeil donné.

Ainsi, le principe de l'invention repose sur l'utilisation astucieuse de moyens optiques semi-réfléchissants configurés pour permettre d'aligner optiquement l'axe optique du capteur d'image sur l'axe de référence correspondant à l'axe de vision de l'œil de l'utilisateur. La disposition externe du capteur d'image et l'obtention d'image dans l'axe de capture grâce aux moyens optiques semi-réfléchissants permettent un relâchement des contraintes optiques, ce qui offre la possibilité d'utiliser des optiques plus simples et moins coûteuses contrairement aux solutions de l'art antérieur, et de meilleure qualité. Le traitement d'image s'en trouve ainsi simplifié.

La présente invention trouve une application particulièrement intéressante dans le domaine de l'orthoptie où les mesures dimensionnelles précises des mouvements des yeux requièrent des images à fort contraste et exemptes de déformation géométrique, ce qui est rendu possible grâce à la configuration astucieuse du système embarqué de l'invention. Par abus de langage, on notera qu'un dispositif comprenant un système embarqué sur un support peut être lui-même appelé dispositif embarqué.

Selon un aspect particulier de l'invention, les moyens optiques semi-réfléchissants comprennent, pour ledit oeil donné, une lame optique semi-réfléchissante inclinée par rapport à l'axe de référence dudit oeil donné d'un angle d'inclinaison compris entre 45 et 80 degrés en fonction de l'axe optique du capteur d'image associé audit oeil donné.

La plage d'angles d'inclinaison dépend de la position de la lame par rapport à la monture, et plus particulièrement par rapport à l'axe du capteur d'image. Par exemple, dans le cas où la lame optique est connectée à la monture par une charnière, la plage d'inclinaison par rapport à l'axe de référence est de 45 à 65 degrés. Dans le cas où la lame optique est déportée de la monture, la plage d'inclinaison par rapport à l'axe de référence est de 60 à 80 degrés.

Selon un aspect particulier de l'invention, la source de lumière est configurée pour émettre une lumière dans une première plage de longueurs d'onde non perceptibles par ledit oeil donné, et le capteur d'image est configuré pour être sensible à une gamme de longueurs d'onde recouvrant au moins en partie ladite première plage. La lame optique semi-réfléchissante est fabriquée à partir d'un matériau optiquement transparent dans une deuxième plage de longueurs d'onde perceptibles par ledit oeil donné, et comprend une première face faisant face à la face externe de la monture, ladite première face étant au moins partiellement traitée en surface pour réfléchir une lumière incidente dans ladite première plage.

La lame optique semi-réfléchissante selon l'invention peut être fabriquée à partir d'un matériau optiquement transparent dans la plage de longueur d'onde du visible, tel que par exemple le polycarbonate, un verre organique ou minéral.

Selon un aspect particulier de l'invention, ladite première face est en outre au moins partiellement traitée en surface pour transmettre une lumière incidente dans ladite deuxième plage.

Un traitement antireflet dans la bande spectrale du visible permet de minimiser les réflexions parasites dans cette bande spectrale (sources potentielles de gênes pour l'utilisateur du dispositif d'oculométrie).

On peut prévoir également que la deuxième face de la lame semi-réfléchissante, opposée à la première face, soit au moins partiellement traitée en surface, comme pour la première face, c'est-à-dire à la fois pour réfléchir une lumière incidente dans ladite première plage et pour transmettre une lumière incidente dans ladite deuxième plage

Selon un aspect particulier de l'invention, la première plage de longueurs d'onde est comprise entre 750 et 3 000 nm et la deuxième plage de longueurs d'onde est comprise entre 400 et 700 nm.

En pratique, la première plage de longueurs d'onde est typiquement comprise entre 750 et 800 nm, correspondant à une plage de longueurs d'onde proches infrarouges.

Selon un aspect particulier de l'invention, la première face comprend une portion de surface traitée, de forme prédéfinie, centrée sur l'axe de référence dudit oeil.

Cela permet de limiter le coût du traitement de surface.

Selon une première implémentation particulière, les moyens optiques semi-réfléchissants comprennent une lame optique semi-réfléchissante, à inclinaison réglable, commune aux deux yeux de l'utilisateur.

Selon une deuxième implémentation particulière, les moyens optiques semi-réfléchissants comprennent un ensemble de deux lames optiques semi-réfléchissantes à inclinaison réglable, chacune associée à un oeil distinct de l'utilisateur.

Selon un autre aspect particulier de l'invention, le dispositif d'oculométrie comprend des moyens de fixation mécanique de la monture aux moyens optiques semi-réfléch issants.

Selon une première mise en oeuvre particulière, la source de lumière associée audit oeil donné est disposée sur la face interne de la monture et dirigée vers ledit oeil donné.

Selon une deuxième mise en oeuvre particulière, la source de lumière associée audit oeil donné est disposée sur la face externe de la monture et dirigée vers les moyens optiques semi-réfléchissants. L'éloignement de la source de lumière et du capteur par rapport à l'œil de l'utilisateur et leur positionnement dans l'axe de vision permet, contrairement aux configurations classiques où la monture intègre une source de lumière et un capteur au niveau de la face interne, de minimiser les déformations naturelles de la pupille et de rendre l'éclairement de l'œil plus homogène.

La source de lumière peut comprendre un jeu d'une ou de plusieurs diodes électroluminescentes. Les diodes électroluminescentes peuvent être régulièrement réparties sur la face interne ou externe de la monture selon l'implémentation choisie. Pour la deuxième implémentation, elles peuvent être régulièrement réparties autour du capteur d'image de sorte que le faisceau de lumière générée se trouve centré autour de l'axe optique du capteur d'image.

Selon un autre aspect particulier de l'invention, la monture comprend au moins un élément de repérage représentatif de l'orientation spatiale de la monture (par exemple des amers visuels).

Cette caractéristique permet d'obtenir des informations sur la position de la tête de l'utilisateur par rapport à une position de référence. Elle permet de s'affranchir de l'utilisation d'une mentonnière par exemple dans le cas d'un test d'orthoptie.

Diverses utilisations du dispositif précité sont possibles, notamment, mais non exclusivement, pour la réalisation d'un oculomètre à usage orthoptique.

### 5. LISTE DES FIGURES

D'autres caractéristiques et avantages de l'invention apparaîtront à la lecture de la description suivante, donnée à titre d'exemple indicatif et non limitatif, et des dessins annexés, dans lesquels :
- la figure 1 est une vue de côté d'un dispositif d'oculométrie selon un mode de réalisation particulier de l'invention ;
- la figure 2 est une vue de face du dispositif d'oculométrie illustré à la figure 1 ;
- la figure 3 est une vue en perspective du dispositif d'oculométrie illustré à la figure 1;
- les figures 4A et 4B représentent le principe schématique du système optique embarqué selon l'invention ;
- la figure 5 est une vue de côté d'un dispositif d'oculométrie selon une variante de réalisation ;
- la figure 6 est une vue en perspective du dispositif d'oculométrie illustré à la figure 5;
- la figure 7 illustre un exemple de réalisation d'une monture intégrant des sources de lumière sur la face interne de celle-ci.

### 6. DESCRIPTION DÉTAILLÉE

Sur toutes les figures du présent document, les éléments et étapes identiques sont désignés par une même référence numérique.

On s'attache plus particulièrement dans la suite de ce document à décrire un dispositif d'oculométrie portatif, objet de la présente, dans le cadre de son utilisation pour l'orthoptie. L'invention ne se limite bien sûr pas à ce domaine particulier d'application, mais présente un intérêt pour toute solution oculométrique embarquée sur un support devant faire face à une problématique proche ou similaire à celle présentée plus haut en relation avec l'art antérieur.

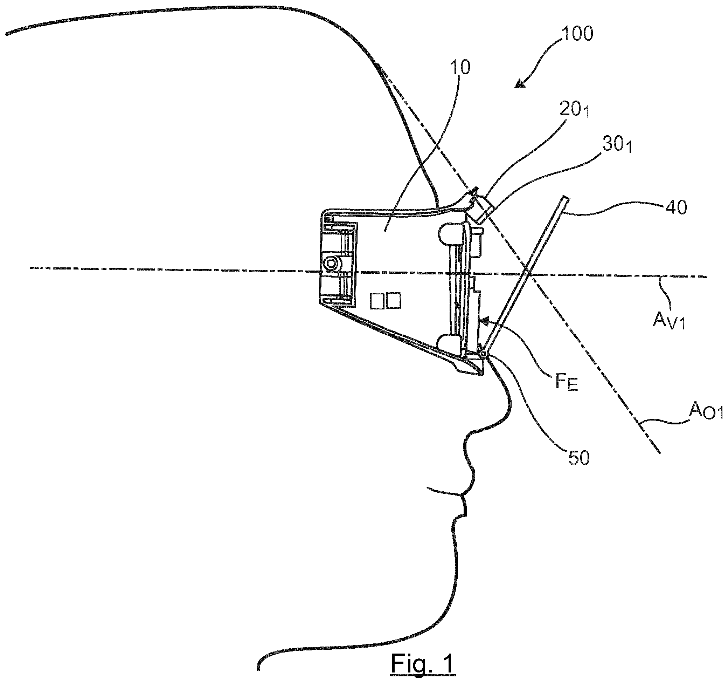

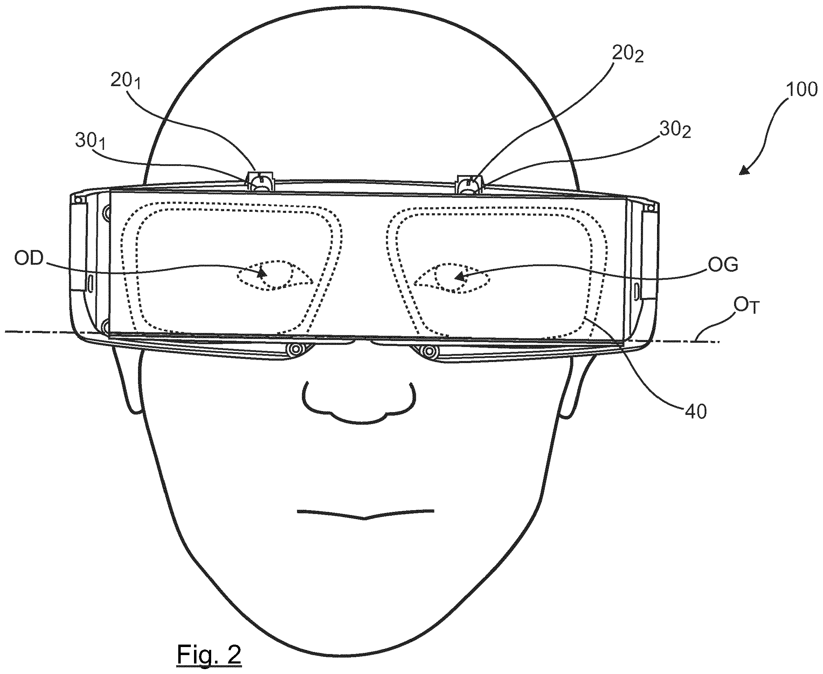

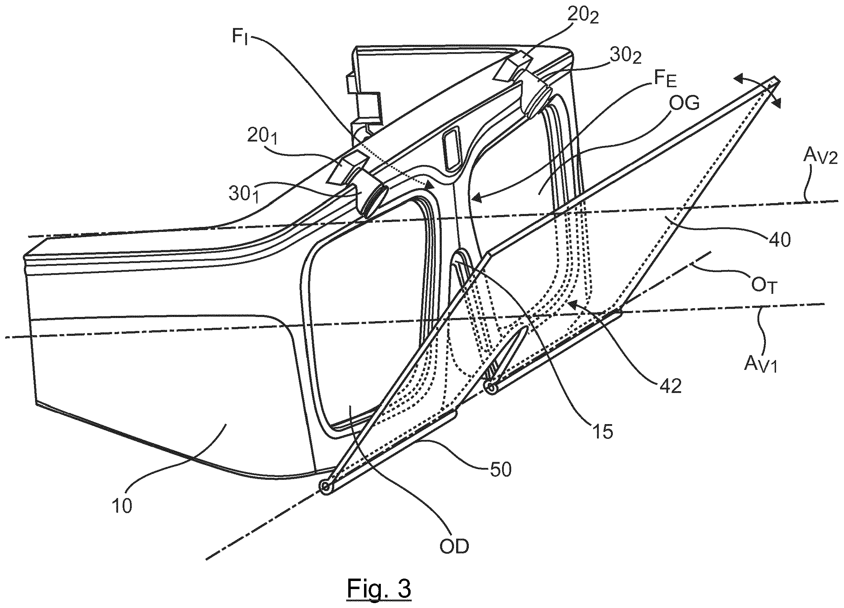

Les **figures 1****,** **2** **et** **3** illustrent différentes vues d'un dispositif d'oculométrie portatif 100 selon un mode de réalisation particulier de l'invention.

Le dispositif d'oculométrie 100 illustré ici comprend une monture (ou support) 10 adaptée pour être portée par un utilisateur.

Cette monture 10 se met sur le visage comme la monture d'une paire de lunettes. Elle comprend deux branches latérales (non représentées ici pour simplifier les figures) adaptées à être chacune montée sur l'un distinct de deux oculaires OD (pour « Œil Droit ») et OG (pour « Œil Gauche ») par l'une de ses extrémités formant charnière. Elle comprend en outre un pont nasal 15 adapté à être monté entre les deux oculaires OD et OG, pour reposer sur le nez. Les deux branches sont de préférence pliables. On pourrait par exemple remplacer les deux branches latérales par un système de cordon se nouant derrière la tête ou tout autre moyen convenable réalisant la même fonction, sans sortir du cadre de l'invention. Une monture sous forme d'un casque de vision binoculaire pourrait être également envisagée sans sortir du cadre de l'invention. Chaque oculaire est configuré pour recevoir un verre optiquement transparent. Mais la présence d'un verre n'est pas obligatoire, et on pourrait très bien prévoir que la monture 10 ne soit équipée d'aucun de verre. On pourrait envisager également la présence d'autres éléments transparents tels qu'un polariseur, un filtre, ou encore un obturateur électro-optique (ou verre actif) pour chaque oculaire de la monture.

La monture 10 présente une face interne F_{I} dirigée vers les yeux de l'utilisateur et une face externe F_{E} opposée à la face interne F_{I}.

Pour la suite de la description, on définit les éléments suivants :
- A_{V1}, l'axe de référence au niveau de la monture correspondant à l'axe de vision de l'œil droit de l'utilisateur (appelé par la suite « axe de vision » par abus de langage);
- A_{V2} l'axe de référence au niveau de la monture correspondant à l'axe de vision de l'œil gauche de l'utilisateur (appelé par la suite « axe de vision » par abus de langage);
- A_{O1} l'axe optique principal du capteur d'image associé à l'œil droit ;
- A_{O2} l'axe optique principal du capteur d'image associé à l'œil gauche.

Le dispositif d'oculométrie 100 selon l'invention comprend un système embarqué sur la monture 10. Ce système embarqué comprend deux systèmes optiques chacun étant associé à un oeil distinct de l'utilisateur, à savoir :
- un premier système optique associé à l'œil droit comprenant :
   * une source de lumière 30₁, par exemple de type infrarouge, destinée à illuminer (au moins partiellement) l'œil droit de façon à générer une lumière réfléchie par cet oeil ;
   * un capteur d'image 20₁, par exemple une caméra du type CCD (pour « *Charged Couple Device* ») ou CMOS (pour « *Complémentary Metal Oxyde Semiconductor »),* destiné à capturer la lumière réfléchie par l'œil droit ;
- un second système optique associé à l'œil gauche comprenant :
   * une source de lumière 30₂, par exemple de type infrarouge, destinée à illuminer (au moins partiellement) l'œil gauche de façon à générer une lumière réfléchie par cet oeil ;
   * un capteur d'image 20₂, par exemple une caméra du type CCD (pour « *Charged Couple Device* ») ou CMOS (pour « *Complémentary Metal Oxyde Semiconductor »),* destiné à capturer la lumière réfléchie par l'œil gauche.

Le capteur d'image 20₁ est disposé spatialement hors axe par rapport à l'axe de vision de l'œil droit A_{V1}. Quant au capteur d'image 20₂, il est disposé hors axe spatialement par rapport à l'axe de vision de l'œil gauche A_{V2}. Il en est de même pour les sources de lumière 30₁ et 30₂ qui sont disposées hors axe respectivement par rapport aux axes de vision de l'œil droit A_{V1} et de l'œil gauche A_{V2}.

Par « disposé spatialement hors axe », on entend, pour un élément donné (capteur ou source de lumière), le fait que cet élément est disposé de manière excentrique par rapport à l'axe de vision de l'œil auquel il est associé, ou autrement dit que cet élément du point de vue structurel n'est pas situé sur l'axe de vision de l'œil auquel il est associé.

Les sources de lumière 30₁ et 30₂ peuvent comprendre chacune un élément lumineux ou un ensemble d'éléments lumineux, comme des diodes électroluminescentes (LED) par exemple. Elles sont configurées pour émettre un signal lumineux dans une plage de longueurs d'onde infrarouges comprise de préférence entre 750 et 850 nm. De manière générale, cette plage de longueurs d'onde doit de préférence couvrir un spectre de longueurs d'onde non perceptibles par l'œil humain.

Dans le présent exemple, la source de lumière 30₁ comprend un jeu de diodes électroluminescentes régulièrement disposées autour de l'axe optique du capteur d'image 20₁ et couplées audit capteur de façon à assurer un éclairage uniforme de l'œil droit et centré sur l'axe de vision A_{V1}. De même, la source de lumière 30₂ comprend un jeu de diodes électroluminescentes régulièrement disposées autour de l'axe optique du capteur d'image 20₂ et couplées audit capteur de façon à assurer un éclairage uniforme de l'œil gauche et centré sur l'axe de vision A_{V2}. En outre, chaque source de lumière 30₁ et 30₂ peut être couplée à une optique de collimation pour collimater les rayons lumineux issus de la source. Ceci permet d'éclairer l'œil de manière plus efficace. Chaque source de lumière peut en outre être couplée à un système d'illumination équipé d'une mire de référence, de forme prédéterminée, destinée à être projetée sur l'œil de l'utilisateur (sans qu'elle soit visible par l'utilisateur), à des fins des tests orthoptiques par exemple.

Chaque capteur d'image 20₁ et 20₂ est configuré pour être sensible à une gamme de longueurs d'onde recouvrant au moins en partie la plage de longueurs d'onde infrarouges précitée (c'est-à-dire de 750 à 850 nm dans présent exemple).

Le principe de l'invention repose sur l'utilisation astucieuse de moyens optiques configurés pour permettre, pour un oeil donné, un alignement optique de l'axe optique du capteur d'image avec l'axe de vision dudit oeil donné, alors que le capteur d'image est spatialement excentré par rapport à l'axe de vision.

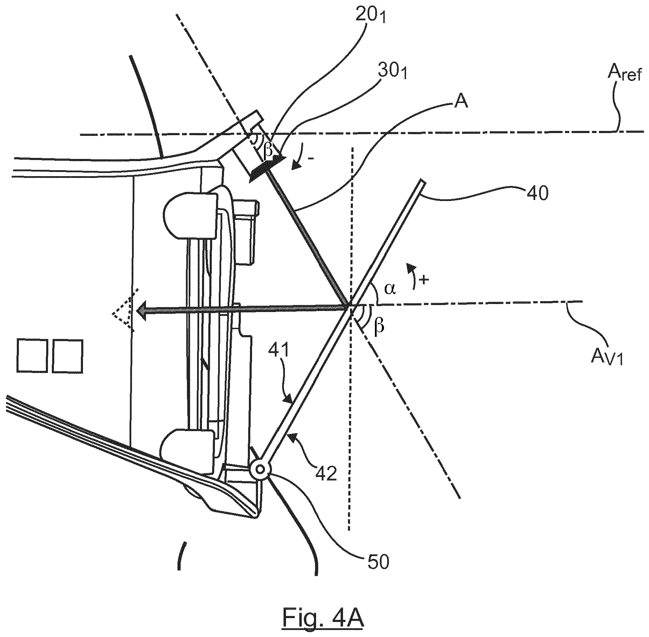

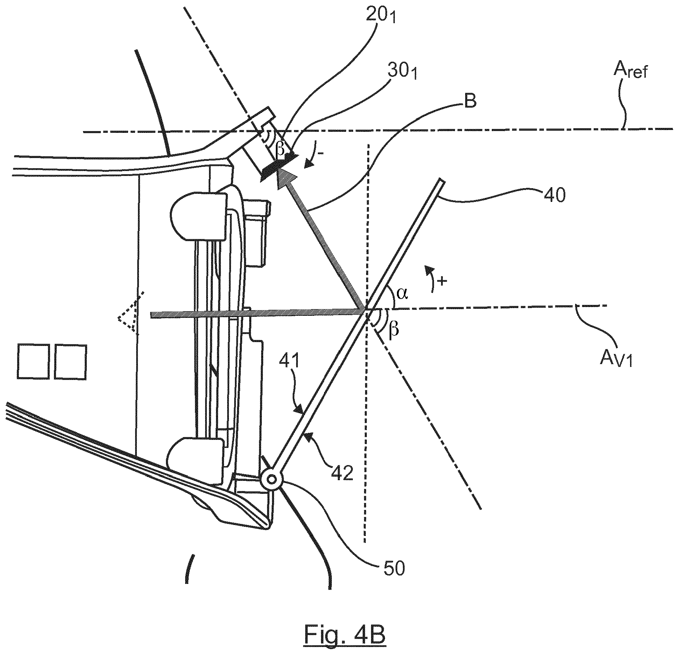

Pour ce faire, les capteurs d'image 20₁ et 20₂ du système embarqué sont disposés sur la face externe F_{E} de la monture 10 et leur axe optique respectifs dirigés vers l'extérieur de la monture 10, comme illustré sur les figures. De plus, le système embarqué comprend une lame optique semi-réfléchissante 40 disposée sur la face externe de la monture et configurés pour réfléchir, d'une part, la lumière réfléchie par l'œil droit directement vers l'axe optique du capteur d'image 20₁ et, d'autre part, la lumière réfléchie par l'œil gauche directement vers l'axe optique du capteur d'image 20₂. Le principe du chemin optique parcouru par les signaux lumineux est décrit plus en détails ci-dessous en relation avec les figures 4A et 4B.

La lame optique semi-réfléchissante 40 (aussi appelée lame séparatrice) est une lame transparente à faces planes et parallèles ayant subie un traitement de surface particulier décrit ci-après.

La lame semi-réfléchissante 40 est fabriquée à partir d'un matériau optiquement transparent dans la bande spectrale du visible (couvrant les longueurs d'onde perceptible par l'œil humain), tel qu'un verre minéral, organique ou en polycarbonate par exemple. La lame semi-réfléchissante 40 comprend plus particulièrement une première face 41, située côté utilisateur, cette première face étant traitée en surface à la fois pour réfléchir la lumière incidente infrarouge (réfléchie par l'œil de l'utilisateur) et pour laisser passer la lumière incidente dans la bande spectrale du visible. Pour se faire, cette première face 41 a subi un traitement anti-réflexion dans la bande spectrale du visible (comprise typiquement entre 400 et 700 nm) de sorte à minimiser les réflexions parasites dans cette bande spectrale et à permettre à l'utilisateur de visualiser l'environnement extérieur à travers la lame optique 40, par exemple une mire de référence projetée sur un écran. La lame semi-réfléchissante 40 comprend également une deuxième face 42 opposée à la première face, qui peut-être traitée en surface la même façon que pour la première face 41.

Le coefficient de réflexion de la lame semi-réfléchissante au niveau de la première face est ajusté dans le domaine infrarouge en fonction de l'angle d'inclinaison choisi pour la lame et de l'orientation du capteur d'image, de façon à minimiser les pertes optiques.

Par ailleurs, les dimensions de la lame semi-réfléchissante 40 sont ajustées de façon à ne pas restreindre le champ de vision de l'utilisateur. Ainsi, la lame semi-réfléchissante 40 doit présenter une surface suffisamment importante de sorte que les bords de celle-ci ne soit pas présentes dans le champ de vision de l'utilisateur (la présence des bords de la lame dans le champ de vision conduit généralement à une gêne visuelle pour l'utilisateur).

On décrit ci-après, en relation avec les **figures 4A** **et** **4B****,** le principe du chemin optique parcouru par les signaux lumineux du système embarqué associé à l'œil droit. Comme illustré sur ces deux figures, la lame semi-réfléchissante 40 est inclinée par rapport à l'axe de vision de l'œil A_{V1} de l'utilisateur d'un angle d'inclinaison α compris entre 45 et 80 degrés. Cet angle d'inclinaison α est choisi en fonction de l'orientation du capteur d'image de telle sorte que l'axe de vision et l'axe optique du capteur soient optiquement confondus. Par exemple, l'angle d'inclinaison α est choisi entre +45 et +65 degrés pour un angle d'inclinaison β de l'axe optique du capteur d'image respectivement entre -90 et -50 degrés par rapport à l'axe de référence A_{ref} qui est associé au capteur 20₁. Cet axe de référence A_{ref} est parallèle à l'axe de vision A_{V1}.

Plus généralement, si l'angle d'inclinaison de la lame est α (sens antihoraire) par rapport à l'axe de vision A_{V1}, l'angle d'inclinaison β du capteur (sens horaire) par rapport à ce même axe de vision A_{V1} est déterminé avec l'équation suivante β = 2 × α - 180

Par contre, si l'on prend la référence par rapport à la perpendiculaire de l'axe de vision de l'œil (représentée par la ligne verticale en pointillés sur les figures), la lame semi-réfléchissante 40 est inclinée d'un angle d'inclinaison compris entre 10 et 45 degrés, et préférentiellement entre 25 et 45 degrés.

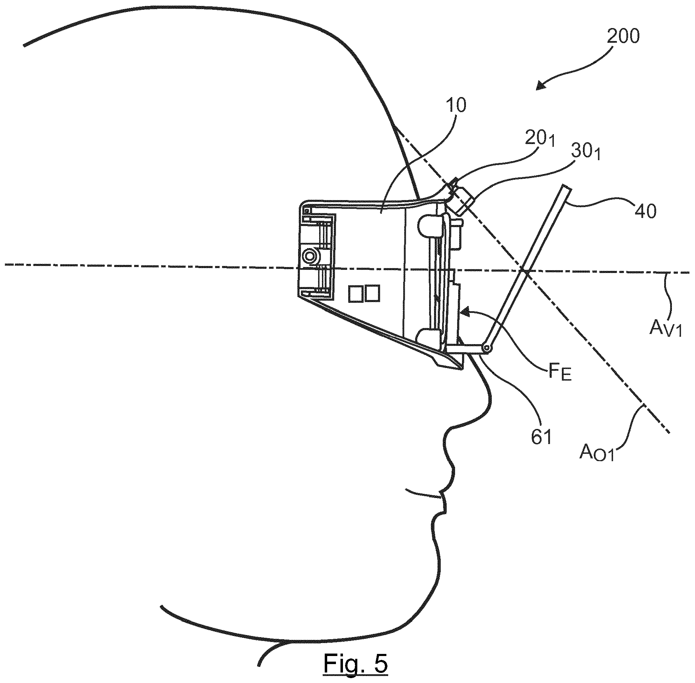

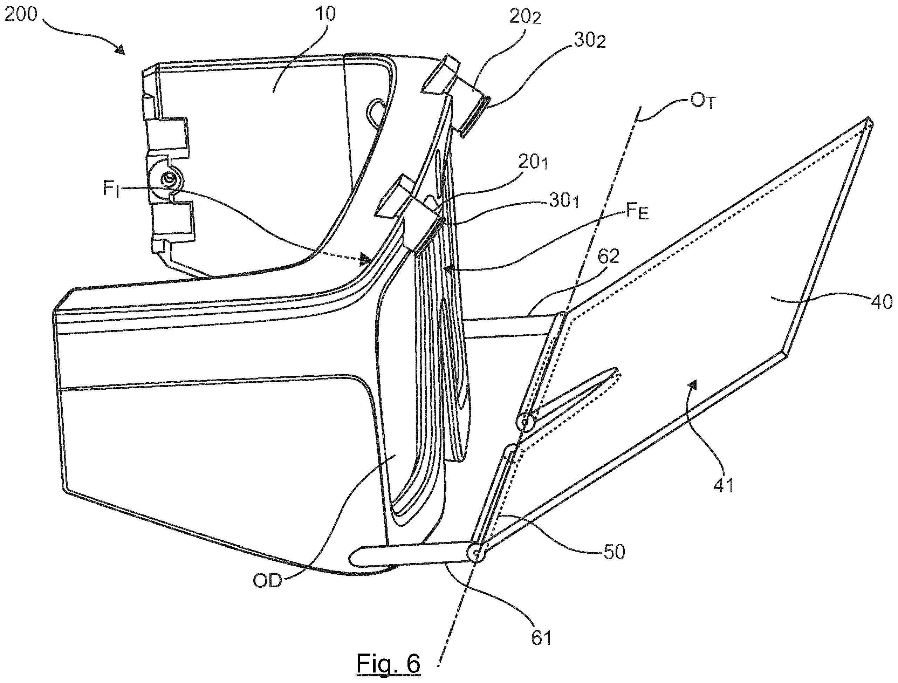

Dans le cas où la lame optique est déportée de la monture comme illustré sur les figures 5 et 6 (dont le principe est décrit plus loin), l'angle d'inclinaison α est choisi entre 60 à 80 degrés.

Cet angle d'inclinaison α dépend de la position du capteur d'image 20₁ sur la monture et l'orientation de son axe optique relative à la lame semi-transparente 40. La lame semi-transparente 40 est configurée pour :
- pour réfléchir, d'une part, la lumière infrarouge générée par la source de lumière 30₁ (par l'intermédiaire de la première face 41) vers l'œil droit de telle sorte que la lumière infrarouge emprunte un chemin optique A coïncidant avec l'axe de vision de l'œil droit A_{V1} (figure 4A) pour la partie comprise entre la lame 40 et l'œil droit, et
- pour réfléchir, d'autre part, la lumière infrarouge réfléchie par l'œil droit (par l'intermédiaire de la première face 41) vers le capteur d'image 20₁ de telle sorte que la lumière infrarouge emprunte un chemin optique B coïncidant avec l'axe optique A_{O1} pour la partie située entre la lame 40 et le capteur d'image 20₁ (figure 4B).

Ainsi, le système embarqué est conçu de façon à aligner optiquement l'axe de vision de l'œil de l'utilisateur sur l'axe optique du système d'imagerie du dispositif. La disposition externe du capteur d'image combinée à l'obtention d'images directement dans l'axe de capture grâce aux moyens optiques semi-réfléchissants a pour effet de permettre un relâchement des contraintes optiques (par rapport aux configurations classiques de l'art antérieur dans lesquelles le capteur d'image est disposée sur la face interne de la monture et donc à proximité de l'œil de l'utilisateur). Cette configuration astucieuse offre ainsi la possibilité d'utiliser des optiques d'imagerie plus simples et moins coûteuses que celles utilisées dans les dispositifs de l'art antérieur, c'est-à-dire des optiques à focales plus longues et moins sujettes aux aberrations que l'on retrouve dans les optiques à grandes ouvertures. Le traitement d'image s'en trouve ainsi simplifier.

Le principe décrit ci-dessus est appliqué au système optique associé à l'œil droit. Ce principe s'applique également et de la même façon au système optique associé à l'œil gauche.

Dans cet exemple d'implémentation, les capteurs d'image 20₁ et 20₂ et sources lumineuses 30₁ et 30₂ sont disposés sur la partie supérieure de la monture 10. Ainsi, la lame semi-réfléchissante 40 est disposée sur la partie inférieure de la monture de façon à permettre que la lumière réfléchie par l'œil emprunte entre la lame et le capteur un chemin optique se superposant avec l'axe optique dudit capteur. Bien entendu, il s'agit là d'un exemple d'implémentation particulier et l'homme du métier pourrait très bien envisager une configuration inverse (dans laquelle les capteurs d'image et sources lumineuses seraient disposés sur la partie inférieure de la monture 10) tout en conservant le même principe que celui exposé ci-dessus sans sortir du cadre de l'invention.

Dans une implémentation particulièrement avantageuse, la lame semi-réfléchissante 40 est à inclinaison réglable de façon que l'angle d'inclinaison α puisse être ajustée précisément par l'orthoptiste en fonction de l'axe optique du capteur d'image qui lui est fixe. La lame semi-réfléchissante 40 est fixée sur la partie inférieure de la monture 10 à l'aide de moyens de fixation mécanique 50, de type charnière par exemple. Ces moyens de fixation mécanique 50 sont conçus de manière à permettre un mouvement de rotation de la lame semi-réfléchissante 40 par rapport à la monture 10 selon l'axe d'articulation O_{T}. Tout autre type de moyens de fixation mécanique réalisant la même fonction (fixation de la lame à la monture et rotation de la lame par rapport à la monture) peuvent être envisagées. Ces moyens de fixation mécanique 50 peuvent également être conçus de manière à permettre un montage et un démontage de la lame semi-réfléchissante 40.

Selon une mise en oeuvre particulière, la lame semi-réfléchissante est inclinable entre deux positions :
- une position de fonctionnement qui est dédiée aux mesures oculométriques (telle qu'illustré sur les figures 1, 3, 4A et 4B) ; et
- une position de repos (typiquement lorsque la lame 40 est disposée à l'horizontal (parallèle à l'axe A_{V1})) ;

Selon une mise en oeuvre particulièrement avantageuse, la lame semi-réfléchissante 40 est traitée en surface pour permettre la transmission de la lumière incidente provenant de l'environnement extérieur dans le champ de vision de l'utilisateur quelle que soit la position de la lame, de telle sorte que l'utilisateur puisse continuellement observer l'environnement extérieur quelle que soit la position de la lame 40.

Le mode de réalisation particulier décrit ci-dessus prévoit la mise en oeuvre d'une lame optique semi-réfléchissante 40 commune aux deux yeux de l'utilisateur. Bien entendu, on pourrait prévoir, dans une variante de réalisation, un système embarqué comprenant un ensemble de deux lames optiques semi-réfléchissantes chacune associée à un oeil distinct de l'utilisateur et chacune conforme au principe de l'invention décrit plus haut. On pourrait également envisager un système embarqué conforme à l'invention mais uniquement mis en oeuvre pour l'un des deux yeux de l'utilisateur.

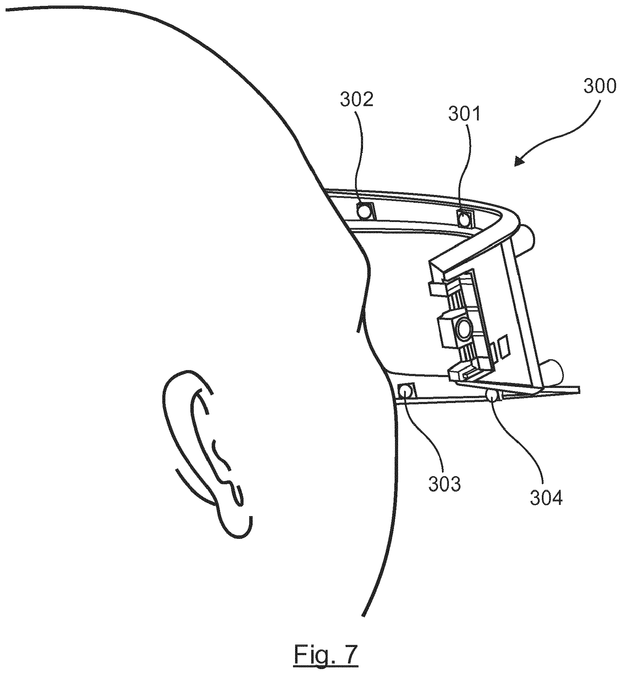

Le mode de réalisation particulier décrit ci-dessus illustre un système embarqué dans lequel la source lumineuse, pour un oeil donné, disposée sur la face externe de la monture du dispositif oculométrique. Il s'agit bien entendu d'un exemple particulier et de nombreux autres modes de réalisation de l'invention peuvent être envisagés. Par exemple, on pourrait envisager, dans une variante de réalisation, de disposer la source lumineuse non pas sur la face externe de la monture, mais sur la face interne de la monture dirigée vers l'œil donné de l'utilisateur, comme illustré sur la **figure 7** par exemple. Sur cette figure, on aperçoit une partie de la face interne d'une monture 300 selon l'invention sur laquelle est fixé quatre diodes électroluminescentes 301, 302, 303, 304 de façon à faire face à l'œil droit de l'utilisateur. Ce nombre est donné à titre indicatif. Un nombre plus important de diodes électroluminescentes et une répartition différente des diodes sur la monture peut être bien entendu envisagés sans sortir du cadre de l'invention, le but étant de permettre un éclairage adapté de l'œil et assurer une mesure oculaire de qualité.

Le système embarqué comprend en outre une unité de traitement, équipée par exemple d'un processeur ou d'un micro-processeur, configurée pour traiter les images issues des deux capteurs d'image 20₁ et 20₂, et générer des informations relatives à la mesure de mouvements oculaires. Cette unité de traitement coopère avec un module de communication sans-fil (ou filaire) permettant de remonter les informations relatives à la mesure de mouvements oculaires vers une unité de calcul (ou CPU pour « *Central Processing Unit* ») situé à distance.

Enfin, le dispositif d'oculométrie peut être équipé d'un ou plusieurs éléments de repérage, par exemple sous la forme de marqueurs (par exemple des amers visuels comme illustrés dans le document d'Emmanuel Zenon, daté du 22 janvier 2004 et intitulé « Localisation topologique, amers visuels et treillis de Galois ») représentatifs de l'orientation spatiale de la monture. Chaque marqueur présente des caractéristiques dimensionnelles de référence connues d'un module de repérage électronique situé à distance. Ce module de repérage est configuré pour déterminer l'orientation spatiale de la monture en fonction de la position réelle des marqueurs dans l'espace. Ceci permet d'obtenir des informations sur l'orientation de la tête du patient de sorte qu'il est possible de s'affranchir de l'utilisation d'une mentonnière dans le cas d'un test d'orthoptie.

Les **figures 5** **et** **6** montrent une vue de côté et en perspective respectivement d'un dispositif d'oculométrie 200 selon une variante de réalisation. Contrairement au mode de réalisation décrite ci-dessus, dans cette variante, la lame semi-réfléchissante 40 n'est pas directement fixée à la monture 10. La lame semi-réfléchissante 40 coopère avec deux bras de rallonge latéraux 61 et 62 eux-mêmes solidarisés de manière fixe à la monture 10 du dispositif. Chaque bras de rallonge comprend à son extrémité des moyens de fixation, de type charnière par exemple, pour la fixation à la lame semi-réfléchissante 40. Ces moyens de fixation sont configurés pour permettre une inclinaison réglable de la lame semi-réfléchissante 40 par rapport à l'axe de la charnière de façon à pouvoir l'orienter convenablement en fonction de la position et de l'orientation du capteur d'image. Ces deux bras de rallonge permettent d'apporter une plus grande liberté dans la recherche d'un compromis entre l'inclinaison de la lame semi-réfléchissante et la position du ou des capteurs d'image. Déporter davantage la lame semi-réfléchissante en avant de la monture permet d'assurer un alignement optique de l'axe de vision de l'œil de l'utilisateur sur l'axe optique du capteur d'image avec un angle d'inclinaison moindre, ce qui est avantageux. En effet, plus la lame optique est inclinée et plus la lame doit présenter une surface importante afin d'éviter que le bord supérieur de la lame soit observable par l'utilisateur (car implique une gêne visuelle).

## Revendications

1. Dispositif d'oculométrie comprenant :
- une monture (10) adaptée pour être portée par un utilisateur, la monture ayant une face interne dirigée vers les yeux de l'utilisateur et une face externe opposée à la face interne,
- un système embarqué sur la monture, comprenant, pour au moins un oeil donné dudit utilisateur :
• une source de lumière (30i, 30₂) destinée à illuminer au moins partiellement ledit oeil donné de façon à générer une lumière réfléchie par ledit oeil donné, et
• un capteur d'image (20i, 20₂) destiné à capturer la lumière réfléchie par ledit oeil donné, ledit capteur d'image étant disposé spatialement hors axe par rapport un axe de référence correspondant à un axe de vision dudit oeil lorsque la monture est portée par ledit utilisateur, le dispositif d'oculométrie étant **caractérisé en ce que** ledit capteur d'image est disposé sur la face externe de la monture et possède un axe optique dirigé vers l'extérieur de la monture, et **en ce que** le système embarqué comprend des moyens optiques semi- réfléchissants (40) disposés sur la face externe de la monture et, pour un oeil donné, configurés par rapport à l'axe de référence dudit oeil donné pour réfléchir directement vers l'axe optique du capteur d'image la lumière réfléchie par ledit oeil donné,
**caractérisé en ce que** les moyens optiques semi-réfléchissants (40) comprennent au moins une lame optique dont la surface est suffisamment grande pour que ses bords n'apparaissent pas dans le champ de vision d'un utilisateur.

2. Dispositif selon la revendication 1, dans lequel la lame optique semi-réfléchissante est inclinée par rapport à l'axe de référence dudit oeil don né d'un angle d'inclinaison compris entre 45 et 80 degrés en fonction de l'axe optique du capteur d'image associé audit oeil donné.

3. Dispositif selon la revendication 2, dans lequel :
- la source de lumière est configurée pour émettre une lumière dans une première plage de longueurs d'onde non perceptibles par ledit oeil donné,
- le capteur d'image est configuré pour être sensible à une gamme de longueurs d'onde recouvrant au moins en partie ladite première plage,
et dans lequel la lame optique semi-réfléchissante est fabriquée à partir d'un matériau optiquement transparent dans une deuxième plage de longueurs d'onde perceptibles par ledit oeil donné, et comprend une première face faisant face à la face externe de la monture, ladite première face étant au moins partiellement traitée en surface pour réfléchir une lumière incidente dans ladite première plage.

4. Dispositif selon la revendication 3, dans lequel ladite première face est en outre au moins partiellement traitée en surface pour transmettre une lumière incidente dans ladite deuxième plage.

5. Dispositif selon l'une quelconque des revendications 3 et 4, dans lequel la première plage de longueurs d'onde est comprise entre 750 et 3 000 nm et la deuxième plage de longueurs d'onde est comprise entre 400 et 700 nm.

6. Dispositif selon l'une quelconque des revendications 3 à 5, dans lequel la première face comprend une portion de surface traitée, de forme prédéfinie, centrée sur l'axe de référence dudit oeil.

7. Dispositif selon l'une quelconque des revendications 1 à 6, dans lequel les moyens optiques semi-réfléchissants comprennent une lame optique semi-réfléchissante, à inclinaison réglable, commune aux deux yeux de l'utilisateur.

8. Dispositif selon l'une quelconque des revendications 1 à 7, dans lequel les moyens optiques semi-réfléchissants comprennent un ensemble de deux lames optiques semi-réfléchissantes à inclinaison réglable, chacune associée à un oeil distinct de l'utilisateur.

9. Dispositif selon l'une quelconque des revendications 1 à 8, la source de lumière associée audit oeil donné est disposée sur la face interne de la monture et dirigée vers ledit oeil donné.

10. Dispositif selon l'une quelconque des revendications 1 à 9, la source de lumière associée audit oeil donné est disposée sur la face externe de la monture et dirigée vers les moyens optiques semi-réfléchissants.

11. Dispositif selon l'une quelconque des revendications 1 à 10, dans lequel la monture comprend au moins un élément de repérage représentatif de l'orientation spatiale de la monture.

## Patentansprüche

1. Augenverfolgungsvorrichtung, umfassend:
- eine Fassung (10), die geeignet ist, von einem Benutzer getragen zu werden, wobei die Fassung eine zu den Augen des Benutzers gerichtete Innenseite und eine zu der Innenseite entgegengesetzte Außenseite besitzt,
- ein in die Fassung integriertes System, das für mindestens ein gegebenes Auge des Benutzers umfasst:
● eine Lichtquelle (30i, 30₂), die dazu bestimmt ist, das gegebene Auge mindestens teilweise so zu beleuchten, dass ein von dem gegebenen Auge reflektiertes Licht erzeugt wird, und
● einen Bildsensor (20i, 20₂), der dazu bestimmt ist, das von dem gegebenen Auge reflektierte Licht zu erfassen, wobei der Bildsensor räumlich außeraxial in Bezug auf eine Referenzachse angeordnet ist, die einer Blickachse des Auges entspricht, wenn die Fassung von dem Benutzer getragen wird,
wobei die Augenverfolgungsvorrichtung **dadurch gekennzeichnet ist, dass** der Bildsensor auf der Außenseite der Fassung angeordnet ist und eine optische Achse besitzt, die nach außerhalb der Fassung gerichtet ist, und dadurch, dass das integrierte System semireflektierende optische Mittel (40) umfasst, die auf der Außenseite der Fassung angeordnet sind und, für ein gegebenes Auge, in Bezug auf die Referenzachse des gegebenen Auges dazu ausgestaltet sind, das von dem gegebenen Auge reflektierte Licht direkt zu der optischen Achse des Bildsensors hin zu reflektieren,
**dadurch gekennzeichnet, dass** die semireflektierenden optischen Mittel (40) mindestens eine optische Platte umfassen, deren Fläche ausreichend groß ist, damit ihre Ränder nicht im Sichtfeld eines Benutzers erscheinen.

2. Vorrichtung nach Anspruch 1, bei der die semireflektierende optische Platte in Bezug auf die Referenzachse des gegebenen Auges um einen Neigungswinkel zwischen 45 und 80 Grad in Abhängigkeit von der optischen Achse des dem gegebenen Auge zugeordneten Bildsensors geneigt ist.

3. Vorrichtung nach Anspruch 2, bei der:
- die Lichtquelle dazu ausgestaltet ist, ein Licht in einem ersten Bereich von Wellenlängen auszusenden, die von dem gegebenen Auge nicht wahrnehmbar sind,
- der Bildsensor dazu ausgestaltet ist, für einen Wellenlängenbereich empfindlich zu sein, der den ersten Bereich mindestens zum Teil überlappt,
und bei der die semireflektierende optische Platte aus einem Material hergestellt ist, das in einem zweiten Bereich von Wellenlängen, die von dem gegebenen Auge wahrnehmbar sind, optisch durchlässig ist, und eine erste Seite umfasst, die der Außenseite der Fassung zugewandt ist, wobei die erste Seite mindestens teilweise oberflächenbehandelt ist, um ein einfallendes Licht in dem ersten Bereich zu reflektieren.

4. Vorrichtung nach Anspruch 3, bei der die erste Seite ferner mindestens teilweise oberflächenbehandelt ist, um ein einfallendes Licht in dem zweiten Bereich zu reflektieren.

5. Vorrichtung nach einem der Ansprüche 3 und 4, bei welcher der erste Wellenlängenbereich zwischen 750 und 3000 nm liegt und der zweite Wellenlängenbereich zwischen 400 und 700 nm liegt.

6. Vorrichtung nach einem der Ansprüche 3 bis 5, bei der die erste Seite einen behandelten Oberflächenabschnitt von vordefinierter Form umfasst, der auf die Referenzachse des Auges zentriert ist.

7. Vorrichtung nach einem der Ansprüche 1 bis 6, bei der die semireflektierenden optischen Mittel eine semireflektierende optische Platte mit verstellbarer Neigung umfassen, die den beiden Augen des Benutzers gemeinsam ist.

8. Vorrichtung nach einem der Ansprüche 1 bis 7, bei der die semireflektierenden optischen Mittel eine Anordnung aus zwei semireflektierende optischen Platten mit verstellbarer Neigung umfasst, von denen jede einem anderen Augen des Benutzers zugeordnet ist.

9. Vorrichtung nach einem der Ansprüche 1 bis 8, die dem gegebenen Auge zugeordnete Lichtquelle auf der Innenseite der Fassung angeordnet ist und zu dem gegebenen Auge hin gerichtet ist.

10. Vorrichtung nach einem der Ansprüche 1 bis 9, die dem gegebenen Auge zugeordnete Lichtquelle auf der Außenseite der Fassung angeordnet ist und zu den semireflektierenden optischen Mitteln hin gerichtet ist.

11. Vorrichtung nach einem der Ansprüche 1 bis 10, bei der die Fassung mindestens ein für die räumliche Ausrichtung der Fassung repräsentatives Markierungselement umfasst.

## Claims

1. Oculometry device comprising:
- a mount (10) suitable for being worn by a user, the mount having an inner face directed towards the eyes of the user and an outer face opposite the inner face,
- a system embedded on the mount, comprising, for at least one given eye of said user:
● a light source (30i, 30₂) intended to at least partially illuminate said given eye so as to generate a light reflected by said given eye, and
● an image sensor (20i, 20₂) intended to capture the light reflected by said given eye, said image sensor being disposed spatially off-axis with respect to a reference axis corresponding to a line of sight of said eye when the mount is worn by said user,
the oculometry device being **characterized in that** said image sensor is disposed on the outer face of the mount and has an optical axis directed towards the outside of the mount, and **in that** the embedded system comprises semi-reflecting optical means (40) disposed on the outer face of the mount and, for a given eye, configured with respect to the reference axis of said given eye, to reflect directly towards the optical axis of the image sensor the light reflected by said given eye,
**characterized in that** the semi-reflecting optical means (40) comprise at least one optical plate, the surface of which is large enough for its edges not to appear in the visual field of a user.

2. Device according to Claim 1, wherein the semi-reflecting optical plate is inclined with respect to the reference axis of said given eye by an angle of inclination of between 45 and 80 degrees according to the optical axis of the image sensor associated with said given eye.

3. Device according to Claim 2, wherein:
- the light source is configured to emit a light in a first range of wavelengths not perceptible by said given eye,
- the image sensor is configured to be sensitive to a range of wavelengths at least partly covering said first range,
and wherein the semi-reflecting optical plate is manufactured from a material that is optically transparent in a second range of wavelengths perceptible by said given eye, and comprises a first face facing the outer face of the mount, said first face being at least partially surface-treated to reflect an incident light in said first range.

4. Device according to Claim 3, wherein said first face is also at least partially surface-treated to transmit an incident light in said second range.

5. Device according to either one of Claims 3 and 4, wherein the first range of wavelengths lies between 750 and 3000 nm and the second range of wavelengths lies between 400 and 700 nm.

6. Device according to any one of Claims 3 to 5, wherein the first face comprises a portion of treated surface, of predefined form, centred on the reference axis of said eye.

7. Device according to any one of Claims 1 to 6, wherein the semi-reflecting optical means comprise a semi-reflecting optical blade, with adjustable inclination, common to both eyes of the user.

8. Device according to any one of Claims 1 to 7, wherein the semi-reflecting optical means comprise a set of two semi-reflecting optical blades with adjustable inclination, each associated with a distinct eye of the user.

9. Device according to any one of Claims 1 to 8, the light source associated with said given eye is disposed on the inner face of the mount and directed towards said given eye.

10. Device according to any one of Claims 1 to 9, the light source associated with said given eye is disposed on the outer face of the mount and directed towards the semi-reflecting optical means.

11. Device according to any one of Claims 1 to 10, wherein the mount comprises at least one registration element representative of the spatial orientation of the mount.
